# EUROPÄISCHE PATENTSCHRIFT

(11) **EP 0 451 792 B1**
(45) Veröffentlichungstag und Bekanntmachung des Hinweises auf die Patenterteilung: **19.03.1997**
(21) Anmeldenummer: 91105616.6
(22) Anmeldetag: 09.04.1991
(51) Int. Cl.: C12N 15/76

(54) **Regulierte Genexpression in Streptomyceten**
Regulated gene expression in streptomycetes
Expression régulée de gènes en streptomycètes

(30) Priorität: 12.04.1990 DE 4011863
(43) Veröffentlichungstag der Anmeldung: 16.10.1991
(73) Patentinhaber: HOECHST AKTIENGESELLSCHAFT, 65926 Frankfurt am Main (DE)
(72) Erfinder: Wohlleben, Wolfgang, Dr., W-4800 Bielefeld (DE); Muth, Günter, Dr., W-4904 Enger (DE); Pühler, Alfred, Prof. Dr., W-4800 Bielefeld (DE); Riess, Günther, Johannes, Dr., W-6230 Frankfurt am Main (DE)

(56) Entgegenhaltungen:
- JOURNAL OF BACTERIOLOGY, Band 171, Nr. 11, November 1989, Baltimore, MD (US); D.S. STEIN et al., Seiten 5768-5775
- JOURNAL OF BACTERIOLOGY, Band 170, Nr. 10, Oktober 1988, Baltimore, MD (US); K.J. KENDALL et al., Seiten 4634-4651
- JOURNAL OF BACTERIOLOGY, Band 169, Nr. 9, September 1987, Baltimore, MD (US); K.J. KENDALL et al., Seiten 4177-4183

## Beschreibung

Die Streptomyceten gehören zwar zu den bedeutendsten Produzenten von Antibiotika, sind aber in genetischer Beziehung noch relativ wenig erschlossen. So ist noch wenig über die Mechanismen bekannt, die die Genexpression in Streptomyceten kontrollieren.

Eine induzierte Expression durch Thiostrepton ist zwar bekannt, aber wegen des hohen Preises und der geringen Löslichkeit in Wasser kommt dessen praktische Anwendung im technischen Maßstab nicht in Betracht.

Es wurde nun gefunden, daß das an sich bekannte kil-kor-System aus dem Plasmid pIJ101 für eine regulierte Genexpression in Streptomyceten genutzt werden kann. Dieses System wurde von Kendall and Cohen, J. Bacteriol. 169 (1987) 4177-4183; 170 (1988) 4634-4651, beschrieben, wobei in der zweitgenannten Literaturstelle die gesamte Nucleotidsequenz des Plasmids pIJ101 wiedergegeben ist. Auf diese Sequenz wird im folgenden Bezug genommen. Es wurde insbesondere gefunden, daß die Regulationsregion des kilA-Gens besonders gut für das erfindungsgemäße Verfahren geeignet ist.

Außerdem ist von Stein et al. (J.Bact. 171: 5768-5775, 1989) bekannt, daß der korB Locus für einen Repressor kodiert, welcher seine eigene Transkription reguliert, genau wie auch die Transkription des kilB Promotors. Desweiteren wurde von denselben Autoren gefunden, daß die korA und tra (=kilA)-Gene von überlappenden, divergenten Promotoren transkribiert werden, die auch vom korA-Genprodukt koreguliert werden. Ebenfalls werden von Stein et al. putative kor-Genprodukt-DNA-Bindungsstellen angegeben.

Die Erfindung betrifft somit ein Verfahren zur regulierten Expression eines Gens in einem Streptomyceten-Wirtsstamm, das dadurch gekennzeichnet ist, daß man als Schalter das kilA-KorA-System nutzt, wobei man das zu exprimierende Gen an die kilA-Regulationsregion koppelt und zur Anschaltung ein temperatursensitives KorA-Protein exprimiert und die Temperatur über dessen Schwellenwert anhebt oder daß man das korA-Gen unter die Kontrolle eines schaltbaren Promotors bringt. Weitere Ausgestaltungen und bevorzugte Ausführungsformen dieser Erfindung werden im folgenden näher erläutert und in den Patentansprüchen definiert.

Ein Teil des erfindungsgemäßen "Schalters" besteht in der Regulationsregion des kilA-Gens, worunter diejenige DNA-Region verstanden werden soll, die durch Anlagerung bzw. Bindung des KorA-Proteins reprimiert wird und die zweckmäßigerweise, aber nicht notwendigerweise, den Promotor des kilA-Gens umfaßt. Diese Region wird in der natürlichen DNA-Sequenz durch die Schnittstellen der Enzyme BglII (beginnend bei Nucleotid Nr. 6160) oder HinfI (beginnend bei Nucleotid Nr. 6027) und NcoI (beginnend bei Nucleotid Nr. 5764) begrenzt, wobei die letztgenannte Schnittstelle auch das ATG-Startcodon des KilA-Proteins(dort tra genannt) einschließt. Die DNA-Sequenz TTGACA der -35-Region des kilA-Promotors beginnt mit Nucleotid Nr. 5975, der Transkriptionsbeginn bei Nucleotid Nr. 5939. In Kenntnis dieser Daten ist es möglich, kürzere bzw. modifizierte DNA-Segmente für die Regulationsregion zu verwenden und gegebenenfalls einen anderen Promotor einzusetzen.

Das reguliert zu exprimierende Gen kann unmittelbar an die überhängende Sequenz entsprechend der NcoI- Schnittstelle anligiert werden. Wenn das erste Kodon nach dem Start mit G beginnt, kann die NcoI-Schnittstelle erhalten bleiben.

Der zweite wesentliche Bestandteil des erfindungsgemäßen "Schalters" ist das KorA-Protein, das als Repressor für das kilA-Gen wirkt. Solange hinreichende Mengen an aktivem KorA-Protein vorhanden sind, ist die regulierte Expression des gewünschten Gens abgeschaltet. Für die Anschaltung gibt es deshalb grundsätzlich zwei Möglichkeiten:
a) Das KorA-Protein wird inaktiviert oder
b) es wird eliminiert.

Die Inaktivierung des KorA-Proteins kann dadurch erfolgen, daß man eine temperatursensitive Mutante dieses Proteins exprimiert und zur Einschaltung des gewünschten Gens die Temperatur über den Schwellenwert anhebt, oberhalb dessen die temperatursensitive Mutante unwirksam wird, d.h. nicht mehr geeignet ist, die Regulationsregion des kilA-Gens zu reprimieren. Die Herstellung solcher temperatursensitiver Mutanten ist bekannt und beispielsweise von Birch et al., J. Gen. Microbiol. 131 (1985) 1299-1303, beschrieben. Derartige Mutationen erfolgen in hoher Ausbeute und die erhaltenen temperatursensitiven Proteine sind mit einem entsprechenden Test im Sinne der vorliegenden Erfindung leicht aufzufinden.

Die Eliminierung des KorA-Proteins ist nach mehreren Verfahren möglich:

Es ist mögiich, das korA-Gen unter die Kontrolle eines schaltbaren Promotors zu bringen. Es ist bekannt, daß die Transkription einer mRNA für eine Lysin-Decarboxylase durch Zugabe von Eisen(III)chlorid zur Kultur reprimiert wird (Schupp et al., Gene 64 (1988) 179-188). Die Zugabe von Schwermetallionen zu einem technischen Fermentationsansatz kann jedoch nachteilig sein, beispielsweise wegen der damit verbundenen Korrosionsgefahr.

Vorteilhafter ist es, das korA-Gen in ein temperatursensitives Plasmid zu integrieren, wobei von dem eigenen Promotor oder von einem anderen, in Streptomyceten wirksamem Promotor Gebrauch gemacht werden kann. Temperatursensitive, in Streptomyceten replizierende Plasmide sind bekannt und beispielsweise von Birch et al. beschrieben. Besonders vorteilhaft sind jedoch Plasmide, die von dem temperatursensitiven Replikon des Plasmids pSG5 Gebrauch machen. Dieses Plasmid ist in der EP-B 0 158 872 bzw. in der US-A 4 880 746 und die Verwendung von pSG5 als temperatursensitives Plasmid in der EP-A 0 334 282 beschrieben. Das Plasmid pSG5 hat einen weiten Wirtsbereich und ist mit einer Reihe von anderen Streptomycetenplasmiden kompatibel. In der EP-B 0 158 201 sind Shuttle-Vektoren beschrieben, in denen von diesen Eigenschaften Gebrauch gemacht wird.

Erfindungsgemäß kann deshalb besonders vorteilhaft das korA-Gen in ein Plasmid eingebaut werden, das das temperatursensitive Replikon von pSG5 enthält, wobei dann das zu exprimierende Gen unter der Kontrolle der kilA-Regulationsregion in ein weiteres Plasmid eingebaut wird, das mit dem pSG5-Derivat kompatibel ist sowie oberhalb der Schwellentemperatur des pSG5-Replikons noch stabil repliziert.

Das korA-Gen wird zweckmäßig als Ahall-BclI-Fragment aus dem Plasmid pIJ101 eingesetzt (die Erkennungssequenz für Ahall beginnt bei Nucleotid 5930, die Erkennungssequenz von BclI bei 6753; die CGCACA-Sequenz der -35-Region des korA-Promotors beginnt bei Nucleotid 5957, die Transkription beginnt bei Nucleotid Nr. 5992).

Wird von der Arbeitsweise mit zwei verschiedenen Plasmiden Gebrauch gemacht, so können beide Plasmide, müssen es aber nicht, Shuttle-Vektoren sein. Es ist jedoch darauf zu achten, daß die beiden Plasmide keine homologen Regionen enthalten, damit störende Rekombinationsvorgänge ausgeschlossen sind.

Das zu exprimierende Gen kann auf einem üblichen Streptomyceten-Expressionsplasmid unter der Kontrolle der KilA-Regulationsregion untergebracht werden. Eine vorteilhafte Ausgestaltung der Erfindung besteht darin, daß das für das gewünschte Protein kodierende Gen, das beispielsweise Proinsulin kodiert, an das Gen für den α-Amylaseinhibitor Tendamistat oder an einen Teil dieses Gens gekoppelt wird. Das dadurch kodierte Fusionsprotein wird aus der Zelle ausgeschleust und kann einfach aus dem Fermentationsüberstand gewonnen werden. Derartige Genfusionen sind beispielsweise in den EP-A 0 281 090 und 0 289 936 beschrieben, wobei in EP-A 0 289 936 mehrere Beispiele für Expressionsplasmide enthalten sind, die Proinsulin enthaltende Fusionsproteine kodieren.

In den folgenden Beispielen wird die Erfindung näher erläutert:

### Beispiele

### 1) Konstruktion des Vektors pKORE2

Das (bei der John Innes Foundation, Norwich, England, erhältliche) Plasmid pIJ101 wird mit Ahall und BclI verdaut und das 830 bp große Fragment isoliert.

Aus dem Plamid pSG5 (EP-B 0 158 872 bzw. US-A 4 880 746) wird das 3,8 kbp große EcoRI-BamHI-Fragment isoliert und mit einem BamHI-Ahall-Linker versehen.

Das handelsübliche Plasmid pUC19 wird mit den Enzymen EcoRI und HindIII verdaut und das große Fragment, das das bla-Gen enthält, isoliert.

Zur Einführung eines in Streptomyceten selektierbaren Markers wird das 1,85 kbp große HindIII-BamHI-Fragment mit dem aphII-Gen aus dem Transposon Tn5 eingesetzt.

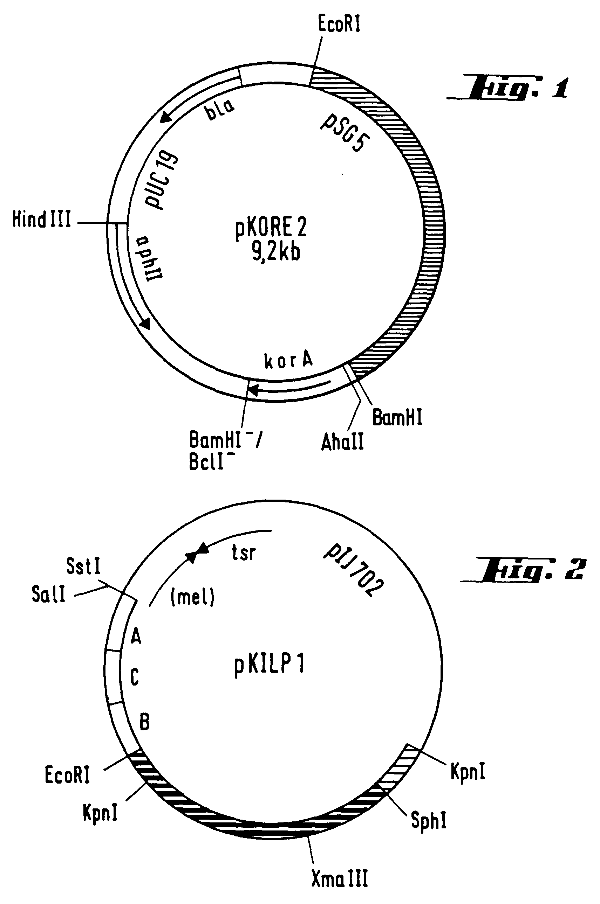

Durch Ligieren dieser DNA-Fragmente erhält man das 9,2 kbp große Plasmid pKORE2 (Fig. 1). Es enthält das temperatursensitive Replikon aus dem Plasmid pSG5 und es repliziert somit stabil nur bei Temperaturen unterhalb 34°C. Bei Temperaturen oberhalb 36°C ist dagegen die Plasmidreplikation inhibiert und das Plasmid wird aus der Zelle eliminiert.

Aufgrund des weiten Wirtsbereichs des pSG5-Replikons und der Kompatibilität mit vielen anderen Vektorsystemen, beispielsweise auf der Basis der Plasmide pSVH1 (EP-B 0 070 522, US-A 4 673 642), pSG2 (EP-B 0 066 701, US-A 4 621 061), pIJ101 (Kieser et al., Mol. Gen. Genet. 185 (1982) 223-238) und SLP1.2 (Thompson et al., Gene 20 (1982) 51-62) kann das Plasmid pKORE2 mit einer Fülle von Plasmiden kombiniert werden, die das gewünschte Protein kodieren.

### Beispiel 2

### Hitzeinduzierte Produktion von Fusionsproteinen

Die Promotorregion des kilA Gens des Plasmids pIJ101 wird mit dem Restriktionsenzym Sau3A ausgeschnitten und in die singuläre BamHI-Schnittstelle des "Promotor-Suchvektors" pIJ 487 (erhältlich bei der John Innes Foundation) kloniert. Nach der Transformation von S. lividans TK24 werden erfolgreiche Klonierungsereignisse an der Expression der Neomycin-Resistenz erkannt. Die isolierte Promotor-Region (Fragment 1) kann nun mit den Restriktionsenzymen Kpnl und SphI ausgeschnitten und durch Elektroelution in bekannter Weise isoliert werden.

Im Plasmid pGF1 (EP-A 0 289 936, ZA 88/3168, AU-A 15 559/88, NZ 222 464) wird durch Ersetzen eines SphI-XmaIII DNA-Fragments durch das verkürzte synthetische SphI-XmaIII DNA-Fragment (SEQ ID NO:1) 
10
(MET)ARG VAL ARG ALA LEU ARG LEU ALA ALA LEU VAL GLY ALA
CGC GTA CGG GCA CTT CGA CTT GCG GCG CTG GTG GGT GCG
G TAC GCG CAT GCC CGT GAA GCT GAA CGC CGC GAC CAC CCA CGC
(SphI)
20
GLY ALA ALA LEU ALA LEU SER PRO LEU (ALA) (ALA)
GGC GCC GCA CTC GCC CTG TCT CCC CTC GC
CCG CGG CGT GAG CGG GAC AGA GGG GAG CGC CGG
(XmaIII)
 der α-AI-Promotor entfernt. Es entsteht das Plasmid pGF1pl, das die Gesamtfusionskonstruktion aus Signalsequenz, αAI-Gen, Proinsulin-Gen und Terminationssequenz enthält. Diese DNA-Sequenz (Fragment 2) kann durch Verdauung mit den Restriktionsenzymen SphI und SstI ausgeschnitten und in bekannter Weise isoliert werden.

Der handelsübliche Expressionsvektor pIJ702 (John Innes Foundation) wird mit den Restriktionsenzymen KpnI und SstI verdaut und das große Fragment (Fragment 3) wird ebenfalls elektroeluiert.

Durch Ligation der Fragmente 1, und 3 wird das rekombinante Plasmid pKILP1 (Fig. 2) erhalten. Nach Transformation von S. lividans TK24, der bereits das kompatible Plasmid pKORE2 enthält, selektioniert man auf die Anwesenheit der Resistenzmarker Neomycin und Thiostrepton. Klone, die beide Plasmide enthalten, sekretieren nach Temperaturerhöhung auf 36°C das gewünschte Fusionsprotein aus dem α-Amylase-Inhibitor und Proinsulin, das in an sich bekannter Weise nachgewiesen und isoliert werden kann.

### SEQ ID NO:1

- ART DER SEQUENZ:: Nucleotid mit entsprechendem Protein
- SEQUENZLÄNGE:: 76 Basen
- STRANGFORM:: Doppelstrang mit überstehenden Erkennungssequenzen
- TOPOLOGIE:: linear
- ART DES FRAGMENTS:: N-Terminus (Signalsequenz)
- HERKUNFT:: synthetische DNA

CGC GTA CGG GCA CTT CGA CTT GCG GCG CTG GTG GGT CGC
G TAC GCG CAT GCC CGT GAA GCT GAA CGC CGC GAC CAC CCA CGC
Met Arg Val Arg Ala Leu Arg Leu Ala Ala Leu Val Gly Ala
1 10

GGC GCC GCA CTC GCC CTG TCT CCC CTC GC
CCG CGG CGT GAG CGG GAC AGA GGG GAG CGC CGG
Gly Ala Ala Leu Ala Leu Ser Pro Leu Ala Ala
20

## Patentansprüche

1. Verfahren zur regulierten Expression eines Gens in einem Streptomyceten-Wirtsstamm, dadurch gekennzeichnet, daß man als Schalter das kilA-korA-System einsetzt, wobei man das zu exprimierende Gen an die kilA-Regulationsregion koppelt und zur Anschaltung ein temperatursensitives KorA-Protein exprimiert und die Temperatur über dessen Schwellenwert anhebt.

2. Verfahren nach Anspruch 1, dadurch gekennzeichnet, daß man das korA-Gen unter die Kontrolle eines schaltbaren Promotors bringt.

3. Verfahren nach Anspruch 1, dadurch gekennzeichnet, daß das korA-Gen von einem temperatursensitiven Plasmid exprimiert und die Temperatur über dessen Schwellenwert angehoben wird.

## Claims

1. A process for the regulated expression of a gene in a streptomyces host strain, which comprises employing as switch the kilA-KorA system, where the gene to be expressed is coupled to the kilA regulatory region, and a temperature-sensitive KorA protein is expressed, and the temperature is raised above its threshold to switch on.

2. The process as claimed in claim 1, wherein the korA gene is brought under the control of a switchable promoter.

3. The process as claimed in claim 1, wherein the korA gene is expressed by a temperature-sensitive plasmid, and the temperature is raised above its threshold.

## Revendications

1. Procédé pour l'expression régulée d'un gène dans une souche hôte de streptomycète, caractérisé en ce que l'on utilise comme commutateur le système kilA-korA, en couplant le gène à exprimer à la région de régulation de *kilA,* et en exprimant, pour l'activation, une protéine KorA sensible à la température, et en élevant la température au-dessus du seuil de celle-ci.

2. Procédé selon la revendication 1, caractérisé en ce que l'on met le gène *korA* sous le contrôle d'un promoteur commutable.

3. Procédé selon la revendication 1, caractérisé en ce que le gène *korA* est exprimé par un plasmide sensible à la température, et on élève la température au-dessus de son seuil.
